(19) Europäisches Patentamt / European Patent Office / Office européen des brevets

(11) **EP 4 556 051 A1**

(12) **EUROPEAN PATENT APPLICATION**

(43) Date of publication:
**21.05.2025 Bulletin 2025/21**

(21) Application number: **23210170.9**

(22) Date of filing: **15.11.2023**

(51) International Patent Classification (IPC):
**A61M 39/28** (2006.01)     **F16K 7/06** (2006.01)

(52) Cooperative Patent Classification (CPC):
**A61M 39/284; F16K 7/063**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Sartorius Stedim FMT**
**13400 Aubagne (FR)**

(72) Inventors:
• **Delasalle, Brice**
**13600 La Ciotat (FR)**
• **Halin, Mickael**
**13600 La Ciotat (FR)**

(74) Representative: **Derambure Conseil**
**c/o Plasseraud IP**
**104 Rue de Richelieu**
**CS92104**
**75080 Paris Cedex 02 (FR)**

(54) **DEVICE FOR PINCHING-OFF HOSES**

(57)     A hose clamp (1) with two passages (31, 32) distributed in two clamping jaws (11, 12) includes two clamping parts (8a, 8b), between which a hose segment of a flexible hose passing through the two passages can be nipped in closed state of the clamp (1). Offset the hose passageway, two clamp ends (E1, E2) have interacting securing elements that anchor the clamping jaws (11, 12) in a closing position, one of these ends (E2) having a securing surface (TS) with at least one retaining rib or tooth facing inwardly, while the other end (E1) is actuatable in response to a pushing action and provided with a tongue (5). Two outer lips (9) are provided at the two respective ends. In expanded state of the clamp, the tongue (5) is transverse to the securing surface (TS) and protrudes outwardly, entirely inside a mouth region (ZD) delimited between the outer lips.

FIG. 1

EP 4 556 051 A1

**Description**

FIELD OF THE INVENTION

The instant disclosure relates to fluid management systems, and more particularly to devices for pinching-off flexibles hoses carrying liquid for the purpose of cutting-off and/or regulating the throughflow, this device possessing two interacting stirrups or jaws, between which the hose is nipped.

TECHNICAL BACKGROUND

Hoses carrying biopharmaceutical fluid are commonly used for interconnecting flexibles pouches or similar parts of a circuit. The term "biopharmaceutical fluid", in the following, is understood to mean a product of biotechnology (culture media, cell cultures, buffer solutions, artificial nutrition liquids, blood products and blood product derivatives) or a pharmaceutical product or more generally a product intended for use in the medical field. In such field, the operators often wear gloves that are likely to be cut when the clamps have one or more protruding edges. The hoses carrying such kind of fluid may be involved for connection to an inlet or outlet of a bioreactor or the like, for instance for single-use applications, in combination with one or more clamping devices.

The clamps are generally designed to provide an efficient pinching-off of such hoses, allowing the through-flow to be partially throttled, or even to be cut off completely.

Document US 6196519 discloses a squeeze clamp that is compact to prevent accidental interaction between different clamps, while being relatively easy to be closed. However, such design of clamp cannot allow a gradual fastening. Moreover, the securing edge protrudes close to/almost in alignment with the upper end surface of the clamp to be pushed with the finger downwardly to cause the snap-locking relationship. As a result, an operator's glove may easily be cut by the protruding edge.

Documents US 6644618 and US 8801677 show clamps provided with anchoring means compatible with a gradually fastening of the clamp ends, using a clamp surface with grooves for engagement of the securing edge. The securing elements of such type, which are quite large, rigid, forming a sharp edge, must be handled with care. They may be not user-friendly on the one hand, and risk of damage to sterile parts is present on the other hand.

More generally, there is still room for improvement regarding constructions of hose clamps.

OBJECTS AND SUMMARY

For improving situation, embodiments of the invention provide a clamping device for regulating the flow of liquid passing through a flexible hose, the device comprising a first clamping jaw and a second clamping jaw that are arranged for gripping the flexible hose therebetween, using two passages (guiding passages) that are distributed at rear and front ends of the clamping device and are forming a hose passageway for receiving the flexible hose (one of the two passages being typically included in/selectively formed by the second clamping jaw),

wherein the clamping device comprises:

-    two clamping parts, between which a hose segment of the flexible hose passing through the two passages can be nipped in closed state of the clamping device, the two clamping parts being distributed in the first clamping jaw and in the second clamping jaw;
-    offset the hose passageway, two clamp ends distributed in the two clamping jaws and provided with interacting securing elements that anchor the clamping jaws in a closing position, wherein one of the two clamp ends includes a securing surface with at least one retaining rib or tooth facing inwardly, while the other clamp end is actuatable in response to a pushing action and provided with a tongue to engage with said securing surface, two outer lips being provided at the two respective clamp ends,

and wherein in an expanded state of the clamping device, the tongue is;

-    transverse (i.e. orientated transverse and offset) to the securing surface;
-    protruding outwardly, while being housed entirely inside a mouth region delimited between the two outer lips.

With such construction, the clamping device may combine advantage of a quick and robust fastening, using an outer tongue in the first clamping jaw actuatable in a natural manner, with a protecting effect in the expanded position of the device. Typically, the lip in the second clamping jaw may protrude sufficiently beyond the securing surface. The securing surface may thus be concealed by the lip of the corresponding clamp end, while the other lip at the pressing side, before actuation to close the clamping device, can create a shifting of the tongue away from the pressing surface. To obtain a closed position/state (with the clamping effect), the tongue is configured to be displaced closer to the hose, along a stroke that leads to an engagement contact with the securing surface that may be formed at a tooted/ribbed section. More generally, the mouth region delimited by the pair of lips may be significantly offset outwardly relative to the securing surface, which allows the tongue to remain closer to the securing surface, in the expanded state, than respective outermost portions of the lips, which are distal from the securing surface.

When having the tongue edge not exposed due to the greater outward extension of the lips distributed in the clamp ends, inadvertent contact of a thumb toward such tongue cannot occur, before and after pinching engagement of the flexible hose. The pair of lips provide a barrier effect, so that the tongue is recessed in the expanded state (the tongue is typically recessed relative to a tangential plane contacting the two lips).

This prevents drawbacks of an exposed sharp edge, likely to damage a component of a sterile circuit or operator's gloves while the clamp is manipulated.

According to embodiments, the two outer lips are each formed by a bulging in a clamp end. The bulging in one of the clamp ends is arranged to form the outermost end at a front face of the clamping device, where the front face of the clamping device is the face, which is:

- is partly delimited by an outer face of the clamp end, called second clamp end, provided with the securing surface at the rear of such outer face,
- orientated at the opposite from a bendable connection of the clamping device (connection where the two clamping jaws are connected).

According to embodiments the two outer lips are each formed by at least one bulging in a clamp end, with the bulging possibly arranged opposite to the securing surface, the clamp end of the second clamping jaw. The two outer lips can be shifted relative to the respective securing elements to face outwardly, the two outer lips being mutually spaced in the expanded state and preferably also in closed position (any one of the closing positions when the securing surface is included in a toothed section).

According to embodiments of the clamping device, one or more of the following particular features are provided:

- in the first clamp end, the tongue is configured to be shifted radially inward relative to a straight member of the first clamp end, which defines an uppermost outer face of the clamping device, with the tongue possibly extending as an aligned extension of another straight member (so-called second straight member, which is parallel to the first straight member and shifted inwardly in the clamping device).
- in the expanded state, the two outer lips sufficiently protrude along same or similar general direction that protruding direction of the tongue, in order to delimit the mouth region of the device beyond the tongue.
- in order to have the tongue entirely housed or concealed inside the mouth region, the tongue provided in the first clamp end may optionally have a protruding extension representing less than a third of the protruding extension (along same direction) of the lip provided at the second clamp end.
- the first clamp end has a protruding extension lower than 6 mm, for instance inferior or equal to 4 or 5 mm, while each of the lip may protrude with an extension at least equal to 9 or 10 mm.

- the first clamping jaw is an upper jaw provide with a pressing surface for actuating the clamping device to obtain a closing position, in response to a downward pression exerted on the pressing surface that is provided in a bulged part of the first clamp end (end provided with the tongue).
- the clamping device is forming a closed loop in the closed position (and in any of the closing positions when having a toothed section), and an open loop in the expanded position,
- the two clamp ends comprise a first clamp end and a second clamp end that are adapted to constitute the two ends of the open loop.
- the second clamp end generally extends upwardly and the lip of the second clamp end protrude in a front direction, to increase thickness and form a bulge protruding a the opposite from the securing surface.
- the first clamping jaw includes a first bulged end part provided with a first lip (of the two outer lips) that may define an outer free end of the first clamp end.
- the second clamping jaw includes a second bulged end part provided with a second lip (of the two outer lips) that defines an outer free end of the second clamp end.
- the first lip has a outer surface including two portions that are facing along different orientations (one being the pressing surface, the other one being a front surface have same general orientation than a second lip outer face, i.e. facing away/opposite from the bendable connection).
- the first lip may have a convex surface with a convex or rounded profile extending between the tongue and a pressing surface of the first bulged end part.

In some options, the anchoring/securing surface can belong to a toothed section. In embodiments with a first clamp end having the tongue and a second clamp end having the toothed section, one or more of the following particular features maybe provided:

- the flexible hose segment passing through the two passages can be gradually squeezed in response to gradually pressing the first clamp end inward (downward typically, as the pressing surface is facing upwards when the device is actuated to reach a closed position).
- they are several closing positions (at least two closing positions, corresponding to grooves of the toothed section when the tongue can be positioned and typically locked).
- the clamping may be an irreversible clamping.
- the mouth region may be adjacent to the anchoring/securing surface.
- the securing elements, which consist of the tongue and a toothed section, are included in a same piece.

- the securing elements, which consist of the tongue and a toothed section (forming the securing surface), are distributed in two pieces that have same developed length, optionally with each of the clamp ends divided in two halves or end parts.
- the securing elements allow maintaining a closed state of the clamping device when mutually engaged.
- in expanded state/position, the tongue is facing along a direction that does not intersect the lip provided in the opposite clamp end (neither the securing surface, nor the associated lip is intersected by a virtual axis or plane extending along the tongue protruding direction.
- the securing elements are adapted to anchor the clamping jaws in at least two closing positions, in which the tongue protruding direction forms a plane or axis that intersects the securing surface.
- the tongue may have a single free edge so that it may protrude (from the first clamp end) in a protruding direction that does not intersect any other part of the clamping device in the expanded state.

Here, the wording toothed section encompasses any configuration with ribs (ribbed section) suitably forming notches for engagement of the tongue with a retaining/locking effect.

In embodiments of the clamping device, following particulars may be provided, separately or in combination:

- the clamp end provided with the tongue has a longitudinal axis that may substantially coincide with tongue protruding direction.
- the tongue, provided with an elongated base joining two opposite sides of the first clamp end, can be slanted (as viewed in a longitudinal cut view) and provided with a rectilinear edge that extends between two rounded side ends of the tongue, so that the rectilinear edge is shorter than the elongated base of the tongue.
- a by-default interspace between the clamping parts, obtained in the expanded state, is greater than any interspace (between such clamping parts) delimited in any one of the closing positions.
- the tongue is configured, in the expanded state, to protrude along the longitudinal axis that is transverse to the toothed section (for instance perpendicular to a generally planar extension/distribution of the securing surface of the toothed section), typically without intersecting the securing surface.
- the bendable connection may maintain, by default, the longitudinal axis of the clamp end of the first clamping jaw along a direction that deviates relative to the opposite clamp end.

The clamping device has a front-rear extension that corresponds to an alignment direction of the passages for the hose. The clamp ends are offset above such front-rear or alignment direction and one of the two lips (adjacent to the tongue) extends higher than the other one, in the expanded state of the device.

With such arrangement with a pair of spaced lips that protrude radially outward relative to an alignment direction of the passages, the operator has contact surfaces that are available when exerting a pressing action to close the clamping device. For instance, the lip of the first clamp end provides a pressing surface that is away from the tongue, for instance shifted above the tongue. Such lip may correspond to a boss that also extends rearwardly from an uppermost region of the clamping device, with a sloped surface that is well adapted for a downward pressing by a thumb to close the device.

The pressing surface may be not aligned (and not parallel) with the tongue, the pressing surface being typically arranged as an outer surface of a boss forming the lip. The tongue may connect a (lower) base region of such boss, thus extends at same level as the base of this boss.

Not only the edge of the tongue is not the sole part protruding away from the flexible hose, but also:

- the pressing surface for the clamping, arranged on the first clamp end, typically at an attachment/bulged end part, is maintaining an operator's thumb/finger (exerting the pressing) away from the tongue for actuating the clamping action;
- two obstacles are formed by the two lips, so that for a movement exerted along the general pressing direction or forward, accidental contact with the tongue edge is prevented.

In some options of construction for the two clamp ends, one or more of the following features may be provided:

- the lip of the first attachment/bulged end part is provided with an angled surface so that the pressing surface extends substantially planar, transverse to a transition surface that forms a spacing, superior or equal to 5 o 8 mm for instance, between the actuation area (at the pressing surface) and the tongue.
- the tongue protrudes (typically in a front direction) from the transition surface, which may be a front surface.
- the transition surface is adjacent to the tongue and may be (optionally) substantially planar to extend generally parallel to the securing surface in a closed sate.
- the toothed section may comprise two or more teeth, distributed along a direction that is extension direction of the toothed section.
- the two clamp ends comprise a first clamp end, which is provided with the tongue and arranged in a thick or bulged part adjacent to the first clamping part, and a second clamp end arranged at a distance from the second clamping part/protrusion.
- the toothed section is facing inwardly in expanded state, preferably by facing in a rearward direction.
- the toothed section is facing, in the expanded state, toward a first passage of the two passages that is provided in the first clamping jaw.
- the tongue protrudes, in the expanded state, substantially perpendicular to a pressing direction (pressing direction allowing the first clamp end to be displaced and engaged onto said toothed section or similar securing/anchoring surface formed on an inner face of the second clamp end).
- the first clamp end is included in an elongated bulged end part that has a longer extension than the second clamp end, so that the tongue is comparatively more spaced from the first passage than the toothed section is spaced from the second passage.
- typically, when a flexible hose extends across the clamping device through the two passages, the tongue for the expanded state can be radially distal from the hose and more distant from the hose than the toothed section.

The lips may be arranged at a top of the front part of the device, with the upper lip provided in the first clamp end arranged rearwardly relative to the other lip. In the expanded state of the clamping device, the tongue in the first clamp end extends at a rear of a tangential virtual plane that is tangent to the two outer lips. It is understood the tongue (having a given width which may equal to the device width) may protrude along a general direction that (virtually) intersects the tangential virtual plane.

In some options, the lip (of the two outer lips) formed in the first clamping jaw defines an uppermost portion of the clamping device (at least in expanded state), while the other lip defines a front free end of the clamping device that protrudes forward; the virtual plane may be a plane tangent to the uppermost portion and the front free end in the expanded state.

In some options for the clamp end provided with the securing surface, in the second clamping jaw, one or more of the following features are provided:

- the clamp end with the securing surface has a thickness which progressively increases with increased spacing from a transition portion (of the second clamping jaw) adjacent to the second passage, whereby a boss is formed adjacent to the securing surface.
- one of the two outer lips, provided in the second clamping jaw, includes the boss.
- each boss may be formed as a layer added on a plastic piece or support that includes the tongue and the securing section (and which possibly delimits an inner circumference of the clamping device in closed position), with the boss being welded, mechanically fastened or overmolded.
- a hollow may be formed between the plastic piece or support and the boss (optionally arranged as an added layer).
- a camber in outer surface of the second clamping jaw may be provided at the transition portion.
- the transition portion may be thinner (with thickness of the transition portion measure substantially parallel to alignment direction of the two passages) than the lip (second lip) of the second clamp end.
- the transition portion may also be narrower than another transition portion of the second clamping jaw, arranged between the second passage and the clamping part of this second clamping jaw (second clamping part).
- the transition portion may be adjacent to the second clamp end.

According to a particular embodiment, the two outer lips are formed as thick ends and/or may have each a thickness superior to 7 or 9 mm, as measured:

- perpendicular to protruding general direction of the tongue, for thickness of the outer lip provided in the first clamp end;
- perpendicular to direction of extension of the securing surface, for thickness of the outer lip provided in the second clamp end.

According to a particular feature, the securing elements are releasable. In some variants, the tongue and the securing section are configured to interlock irreversibly (thus without ability to re-obtain the expanded state, unless breaking part(s) or irreversibly deforming the device), the device being a single-use device. In any case, the first clamp end and the second clamp end may be spaced apart in the expanded state, which is an open state of the clamping device. The outer lips may be rigid.

Possibly a convex surface (surface of convex profile as seen along a longitudinal plane of the device) of one of the outer lips is facing a slanted portion of the tongue, in the expanded state. This convex surface may be included in a lip with a continuous rounding, covering an angular sector superior or equal to 150°. The convex surface may join the securing surface at one end thereof, while the other end may be facing in opposite direction.

In the clamping device, the first clamping jaw has an outer face provided with a first camber arranged between:

- a first passage amongst the two passages,
- and a first outer boss of the clamping device that is adjacent to the tongue.

Additionally or independently of the specific location of the first camber, the second clamping jaw may provide a second camber in an outer face of the second clamping jaw. The second camber can be arranged between a second passage amongst the two passages and a second outer boss of the clamping device that is provided in the second clamp end.

Typically, the second outer boss is facing in a direction opposite to the securing/anchoring surface (thus not facing the toothed section, but in opposite direction).

The first camber and the second camber provide each an outer recess, for instance with an outer bending, so that each of the clamp ends are deviated (upwardly for the first clamp end and forward for the second clamp end). The deviation angle at each of these cambers may optionally correspond to a slight deviation (for instance with an angle greater than 145° and lower than 180° between the two outer surfaces separated by the camber).

In some options, the toothed section is provided in the first clamping jaw and comprises teeth distributed and mutually spaced along a given alignment direction, wherein the following relation is satisfied: $0.3 \leq L1/L2 \leq 0;6$

where L1 is the spacing distance, measured along the alignment direction, between the toothed section and the tongue in the expanded state, while L2 is the spacing distance, measured along the alignment direction, between the toothed section and an outer surface portion formed in a boss of the first clamping jaw.

The clamping device can be provided with a bendable connection, typically arched, which forms a common end for the first clamping jaw and the second clamping jaw. These jaws are thus bendably connected with each other at this common end and can essentially extend:

- above the hose passageway, for the first jaw;
- and below the hose passageway, for the second jaw.

The securing elements are provided in the two clamp ends, respectively at the opposite of the common end.

According to a first kind of construction, the clamping device is made of a single piece with the bendable connection formed in a C-shaped portion that extends between the two clamping parts. For instance, the clamping device is a one-piece device made of plastic material, using injection molding technology. Increase of thickness in the first clamp end may be obtained by forming an end of annular or rectangular profile, this profile being viewed in a longitudinal plane intersecting the ends (clamp ends and common end) of the jaws.

According to a second kind of construction, the clamping device is made of two pieces provided with interlocking elements, which may optionally be arranged (selectively/exclusively) in the thickness of the clamping device.

The bendable connection and delimitations of the two passages are distributed in the two pieces, so that the two pieces can be fastened using the interlocking elements. Such fastening cab be performed to obtain the clamping device once the flexible hose is already extending between the two pieces.

When having two initially separate pieces, one or each of the two outer lips can be made of two separable lip parts distributed in the two pieces.

In order to ensure a clamping, the pair of clamping parts include, in a first clamping part, a first inwardly-projecting contacting member and, in a second clamping part, a second inwardly-projecting contacting member opposed to the first inwardly-projecting contacting member. The first and second contacting members, each having an inner face of convex profile, are configured to occlude the hose segment of the flexible hose closed state of the clamping device, when the flexible hose extends across the clamping device in alignment with a direction of the two passages.

The first clamp end can constitute or be provided in a hollow section that extends substantially parallel to a tongue protrusion direction, the hollow section being:

- elongated, extending between the tongue and the first clamping member, and
- preferably provided with an outer camber at the opposite from the first clamping member. More generally, at least one amongst the two clamp ends is provided with a hollow, which may optionally open laterally outwards (for instance on both sides, i.e. left and right sides).

BRIEF DESCRIPTION OF THE DRAWINGS

The figures of the drawings are now briefly described.

Fig. 1 is a perspective view of a first embodiment, in expanded state, of a device suitable for pinching-off a flexible hose carrying fluid.

Fig. 2 is a side view of the clamping device of Fig. 1, in a closed position with two clamping parts allowing the pinching of the flexible hose received in aligned passages of the device.

Fig. 3 is a perspective view of a tubing set for circulation of a biopharmaceutical fluid, for instance blood or similar fluid containing living cells or biological compounds, including a flow control clamping device according to an embodiment.

Fig. 4 is a detail view of an embodiment of the clamping device, showing location of the tongue that does not protrudes beyond lips formed at the two ends of the clamping device.

Fig. 5 is a perspective view of a second embodiment, in expanded state, of a device suitable for pinching-off a flexible hose carrying fluid, using two pieces or halves.

Fig. 6 is a top view of the clamping device of Fig. 5, showing the tongue and a top of a protecting protrusion/boss that increases thickness of the attachment end including the tongue.

Fig. 7a schematically illustrates a half that can be assembled with a complementary half, along a longitudinal junction plane, using interlocking means to form the clamping device of Fig. 5.

FIG. 7b illustrates the other half, suitable to be assembled with the half of Fig. 7a.

MORE DETAILED DESCRIPTION

A detailed description of several embodiments of the invention is provided below, accompanied with examples and with reference to the drawings.

In the various figures, the same references are used to designate identical or similar elements. Thickness size is to be understood in a usual way for the skilled person (thickness can be measured in a longitudinal plane/longitudinal cut view of the clamping device), while width reflects transverse size (along a right-left direction) measured perpendicular to such longitudinal plane.

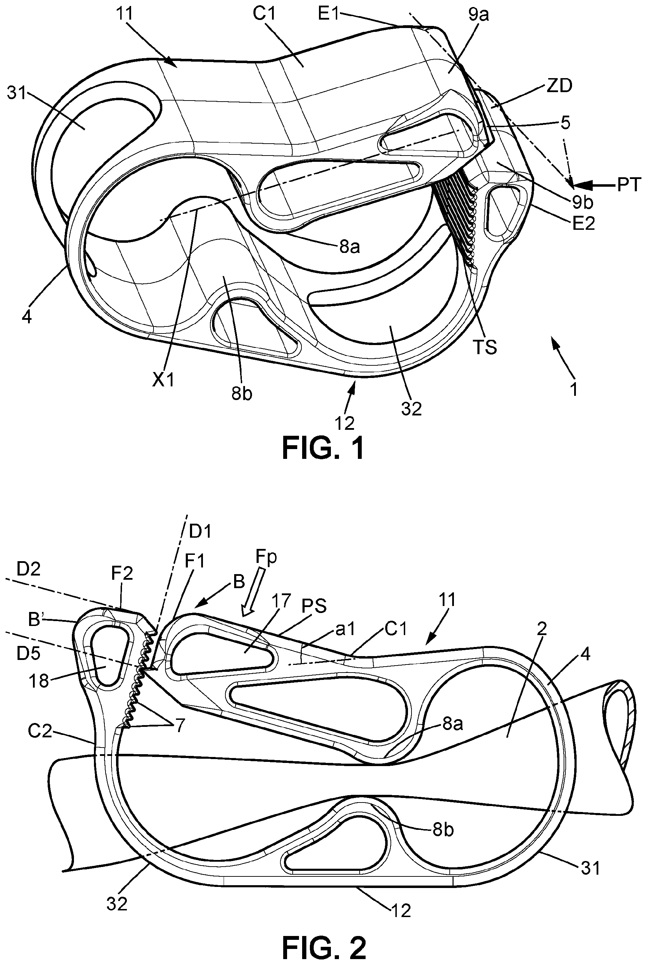

Referring to Figs 1 and 2, it is shown exemplary construction of a hose clamping device 1, here made of a single piece of plastic material. The device 1 may comprise two portions with arched profile, each opened to delimit a passage 31, 32 for a flexible hose 2. A rigid portion, so called lower portion, interconnects such two opened portions and includes an inner projection (inwardly-projecting contacting member). The inner projection, which may have a rounded relief, is forming a clamping part 8b suitable to interact with a complementary clamping part 8a (also having a inwardly-projecting contacting member) provided in a clamping jaw 11 or similar movable part of the device 1.

The device 1 may typically have two clamping jaws 11, 12 and can be actuated from an expanded state, which may be an open configuration, to reach a closed state where the clamping parts 8a, 8b allow a clamping effect, with the hose segment of a flexible hose 2 nipped (such hose segment passing through the two passages 31, 32, as illustrated in Fig. 2).

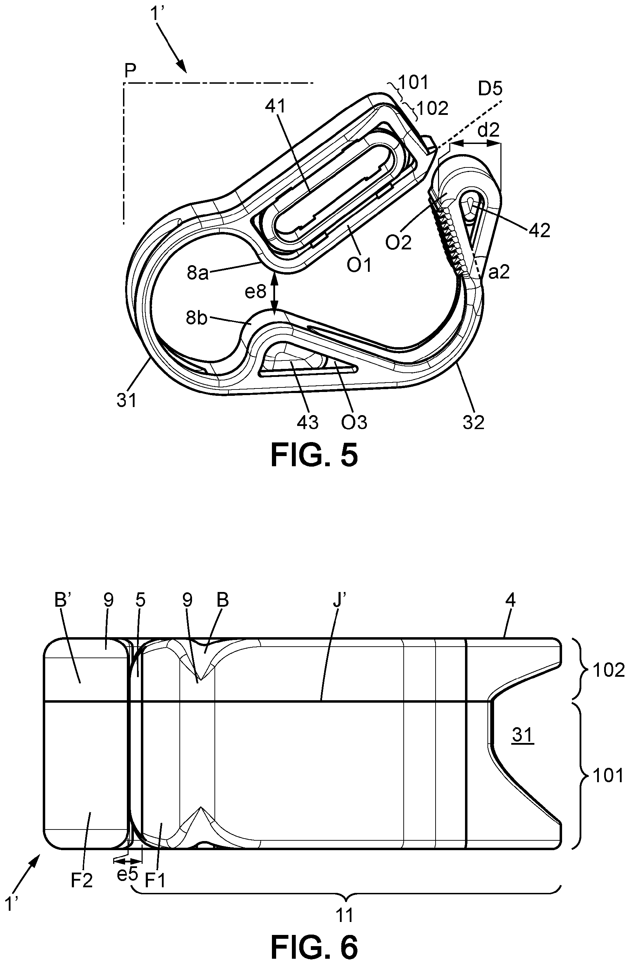

In other embodiments, for instance using a two-piece construction, same parts may be provided so that the clamping device 1' includes two clamping jaws 11, 12. Figs 5, 6 and 7 illustrate such a construction. More generally, geometry of the clamping device 1, 1' may be chosen to have the two passages 31, 32 separated by a region where a pinching can occurs due to the clamping parts 8a, 8b. The two passages 31, 32 may be distributed at rear and front ends of the clamping device 1, 1'. The width (along a left-right direction) of the clamping device 1, 1' may be optionally constant, to obtain two opposite sides or side faces. The device 1, 1' may thus be delimited between two parallel longitudinal planes and a symmetry plane can be provided. More generally, the clamping device 1, 1' has a longitudinal extension, parallel to a hose passageway formed using the two passages 31, 32. Two clamp ends E1, E2 are provided with an offset relative to the hose passageway.

Of course, the clamping device can be any suitable assembly of different/several parts made separately and then fastened.

The two passages 31, 32 may be distributed in the two clamping jaws 11, 12 that are bendably connected with each other at one end thereof, which may be a common end forming the bendable connection 4. The clamping jaws 11, 12 also carry interacting securing elements at the opposite end thereof. A one-piece-construction may be obtained, for instance with the single-piece having a width substantially constant. The width is here measured along a left-right direction, perpendicular to a longitudinal plane of the clamping device 1, 1'. The bendable connection 4, which forms the common end for the first clamping jaw 11 and the second clamping jaw 12, may be relatively thin, typically thinner than thickness in the two clamp ends E1, E2 that include the securing elements. The first clamp end E1, provided in the first clamping jaw 11, has a tongue 5 that protrudes in a direction away from the bendable connection 4. The tongue 5 does not protrude laterally, so that the clamping device 1, 1' may have two sides without any protruding relief. The tongue 5 locally protrudes externally and can be spaced from the second clamp end E2 in an open loop configuration of the clamping device 1, 1' that can correspond to the expanded state. The clamping devices 1 and 1', shown in Fig. 1 and 5, have such expanded state.

The projecting parts 8a, 8b are spaced and have a by-default interspace e8 between these parts, which is obtained in the expanded state. Of course, the spacing size corresponding to the interspace e8 may be measured along a direction, called height direction. Such direction transverse to the hose passageway here corresponds to height, knowing that the first clamping jaw 11 carrying the tongue 5 is typically/conventionally considered as an upper jaw, as in the disposition shown in Fig. 2 and in Fig. 5. Indeed, when the device 1, 1' is taken in hand by an operator, actuation of the clamping device 1, 1' is done when having a top outer face of the device 1, 1' corresponding to outer face of the first clamp end E1 (clamp end provided in the first clamping jaw 11).

The upper jaw outer face provides a pressing surface PS for displacing this first clamp end E1 according to a downward movement. The pressing surface PS may be substantially as wide as the clamping device 1, 1' and/or the pressing surface PS extends from the left side to the right side of the first clamping jaw 11.

For a natural position of the hand, the actuation is performed, by a downward pressing of the first clamp end E1 (for instance using a thumb) to reach an engagement of the tongue 5 against a securing surface, possibly constituted by a toothed section TS, provided in the second clamp end E2. It is understood the clamping device 1, 1' can be actuated by one hand only, the device 1, 1' being operated like a (relatively small) office stapler for reaching a closed state/position.

As well apparent in Fig. 1, the interspace in expanded state is greater than any interspace delimited in any one of the closing positions, between the clamping parts 8a, 8b, when the tongue 5 has been engaged with the toothed section TS due inward movement of the clamp end E1. It can be seen the tongue 5 has a width significantly greater than its protruding extension, which is an extension as measured along the longitudinal axis X1 of the first clamp end E1.

A first lip 9a is provided in the first clamp end E1, in a region away from the securing surface in the expanded state. Along a front-rear direction, it is apparent the protruding extension of the tongue 5 is short with the tongue 5 not extending beyond the front part of the clamping device 1, which is a part formed by a second lip 9b that participates to increase thickness of the second clamp end E2. The second lip 9b is formed outwardly in the clamp end E2, thus at the opposite from the toothed section TS or similar securing surface interacting with the tongue 5. With such arrangement of outer lips 9 at the two respective clamp ends E1, E2, the tongue 5 is not protruding beyond any tangential plane contacting the two clamp ends E1, E2. Non limiting embodiment of Fig. 1 illustrates a tangential plane PT contacting the first lip 9a at an outer surface thereof and contacting the second lip 9b also. When considering the mouth region ZD delimited between the first lip 9a and the second lip 9b, which is a region extending on one side (inner side) relative to the tangential plane PT, it is well apparent the tongue 5 is housed entirely inside the mouth region ZD. As a result, the tongue 5 may locally protrude for the anchoring action, without protruding beyond the mouth region ZD and thus without being reachable in any available tangential orientation, in the expanded state.

At least one amongst the first lip 9a and the second lip 9b may be included in an outer layer of plastic that is curved to form a curved outer surface. Each layer of plastic, forming a clamping device outer surface may be:

- provided with an arcuate shape/profile, possibly with a bridge structure including two bridge sections separated by a hollow space, only the ends of the bride sections joining the remainder of the clamping device 1; and/or
- made of plastic or resilient material less rigid than plastic material of the supporting clamp piece/assembly that includes the two clamping parts 8a, 8b and delimits inner circumference of the clamping device 1, 1'.

The outer lips 9 may include or consist of rubber or elastomer material, in some options.

Referring to Fig. 2, in the second clamp end E2, the second lip 9b is not overlapping the securing surface or toothed section TS at the upper access side thereof. The second lip 9b may be constructed as a boss B' extending only in front of the securing section that is facing rearward (and inwardly). The second clamp end E2 may have a thickness which progressively increases with increased spacing from a lower end of the securing surface, here with increased spacing from a transition portion (of the second clamping jaw 12) adjacent to the second passage 32. The boss B' is formed adjacent to the securing surface, possibly with a hollow present at the second clamp end E2 for increase spacing between the front part of the boss B' and the securing surface, while optimizing amount of material (plastic material typically) used in such end E2.

The two outer lips 9 may have relatively large curvature radii for each outer surface that extends from, respectively:

- from the tongue 5, for the curved outer surface provided in the first lip 9a,
- from an edge of the securing surface that is proximal to the tongue in the expanded state, for the curved outer surface provided in the second lip 9b.

In the first lip 9a, the outer surface may have an upwardly extending front area (at face F1) and an upwardly facing area corresponding to the pressing surface PS (as well apparent in Fig. 2 for instance, forming a part of the device outer circumference in closed state), possibly forming an angled outer surface.

In the second lip 9b, the outer surface may have an upwardly facing front area (at face F2, possibly more or less aligned with the pressing surface PS in closed state) and a front area (as well apparent in Fig. 2 for instance), possibly forming another angled outer surface. The outer surface of the second lip 9b represents another part of the device outer circumference in closed state. In expanded state, an inner space or volume delimited by the clamp opens upwardly through the gap existing between the two clamp ends E1, E2. As apparent in Fig. 1 or Figs 4-5, the gap may be narrower in a region that is delimited by the second lip 9b (near/adjacent to rib(s) 7 of the securing surface) and the tongue 5, the first lip 9a extending away from such narrow gap formed as subpart of the mouth region ZD.

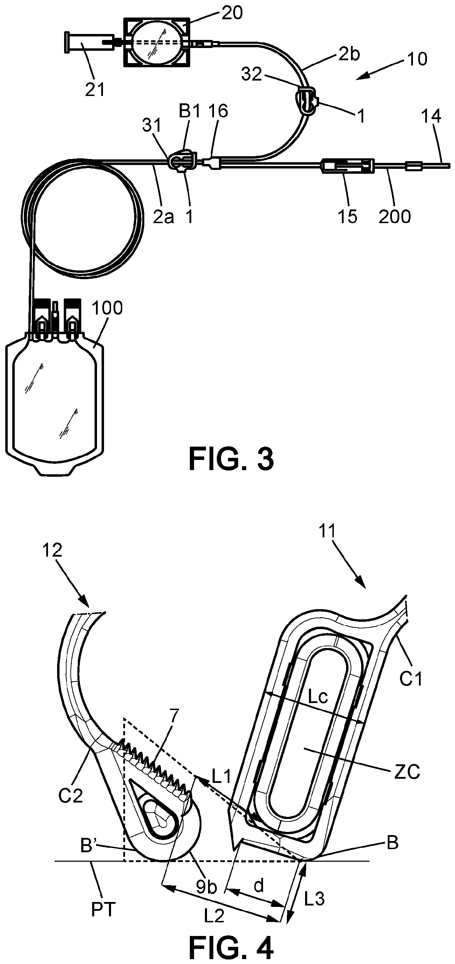

Now referring to Fig. 4, it can be seen that shortest distance L1 in the expanded state between the toothed section TS or rib(s) provided on inner side of the second clamp end E1 and the tongue 5 is less than maximal thickness provided in the first clamp E1 and less than maximal thickness L3 provided in the second clamp E2.

Alternatively or in addition, the following relation can be satisfied: $0.3 \leq L1/L2 \leq 0;6$
where L1 is the spacing distance (shortest distance), measured along the alignment direction, between the toothed section TS and the tongue 5 in expanded state, while L2 is the spacing distance, measured along the alignment direction D1 of the teeth or ribs 7, between the toothed section TS and an outer surface portion (upper portion that may be the uppermost portion of the clamping device) formed in a boss B of the first clamping jaw 11, at the first clamp end E1.

In expanded state of the clamping device 1, 1', the tongue 5 is transverse to direction D1 (thus being transverse to the securing surface) and protrudes along a general direction that may extend intermediate between the outer lips 9. The lips 9 or bosses B, B' may be each bulged in a common direction, for instance direction perpendicular to the tangential plane PT as shown in Fig. 4. This plane PT thus extends farther from the tongue 5. Besides, a hollow ZC may be provided in the first clamping jaw 11 to have the first lip 9a, formed by the boss B, provided with an angled surface that is located much higher than the tongue 5, for instance at least 7 or 9 mm higher (distant along direction D1), thus deviating the tangential plane PT (more vertical when considering the operational position such as illustrated in Fig. 5) as compared to situation where a plane can be tangent at the tongue 5. More generally, a maximum thickness of the first clamp end E1, measured between the pressing surface PS and the lower side of the tongue 5 (thus along direction Fp of pressing as shown in Fig. 2) can be at least three times greater than the protruding extension e5 of the tongue 5.

Of course in the first clamp end E1, the protruding extension e5 of the tongue 5 along direction D5 (protruding direction) is sized to be significantly less than respective extensions of the lips 9 in such direction.

Referring to Fig. 2, the hose passageway extends along a longitudinal rear-front direction of the clamping device 1, with the second clamp end E2 being at the front of the device 1. As shown in Figs 2 and 4, it can be seen the bulge or boss B in first clamp end E1 belongs to a hollow arrangement whose upper face defines the pressing surface PS. The hollow arrangement may have a profile, as considered in a side view, with two long sides (for instance with a lower side extending to the clamping part 8a formed at a rear end thereof, while the upper side is extending rearward from the boss B to a camber region C1) and two short sides that are comparatively shorter. The elongated hollow arrangement may thus have a thickness Lc or radial extension, as measured between the pressing surface PS and the lower side face facing the second clamping jaw 12, which is greater than protruding extension e5 of the tongue 5.

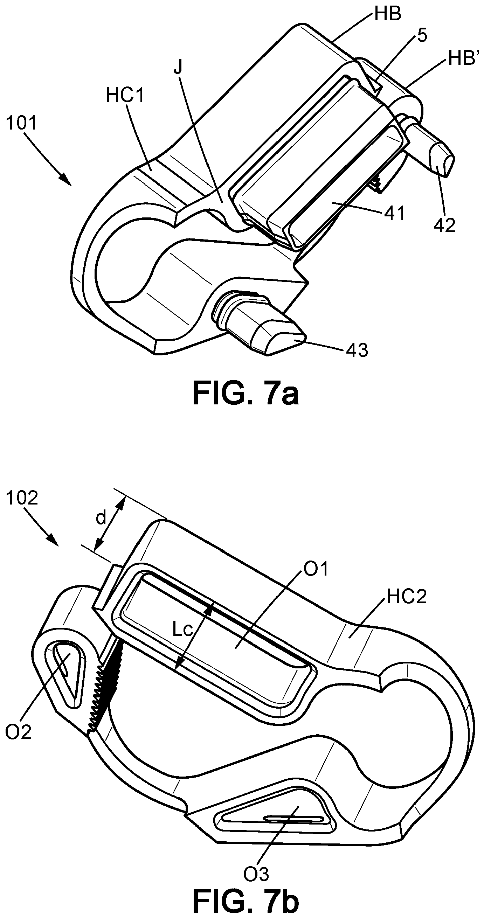

The hollow arrangement may be a connecting arrangement when having a clamp body split in two body parts or pieces 101, 102, as illustrated for instance in Fig. 6 and 7a-7b. Whatever the construction of the body of the clamping device 1; 1', the first clamp end E1 may have a significant extension size d above the protruding tooth or tongue 5, which is a height extension. More generally, the clamp end E1 forms a first lip 9a that defines an uppermost end of the device 1, 1', while the second lip 9b (typically with a front boss B') is forming/arranged as a front end of the device 1, 1'. In expanded state, the tangential plane PT that is tangent to the uppermost end and front end (at lips 9) of the clamping device 1, 1' delimits the mouth region ZD. It is understood that area where the tongue extends in expanded state may be an area substantially in a middle of the mouth region ZD, i.e. between the lips 9 and simultaneously spaced from the toothed section TS and from the angled portion of the boss B forming the uppermost end of the device, for instance with the tongue 5 equidistant between the toothed section and the uppermost portion of the device 1, 1'.

In some embodiment, the relationship d/Lc ≥ 0.5 may be verified (possibly with d/Lc < 0.9), as apparent in Fig. 4 and Fig. 7b for example. The first clamp end E1 may optionally be provided with a C-shaped or U-shaped section due to the hollow 17, ZC that separates an upper branch allowing forming the pressing surface PS from a lower branch that extends toward the first clamping part 8a.

The angled surface at the boss B, possibly with a rounding, may be formed to have face F1 deviating of about 90° relative to general direction D2 of the first clamp end E1 in a closed state (see Fig. 2 for instance). This allow the face F1 to be parallel to and directly facing the toothed section TS, in such closed state, so that the boss B does not interfere with downward movement of the tongue 5 arranged to protrude at a lower end of this face F1.

Each of the clamping jaws 11, 12 may have an outer face provided with camber C1, C2, such cambers C1, C2 being adjacent to bulged parts of these jaws, so that they are adjacent to a transition section where thickness in the jaw 11, 12 greatly varies (increases). Also, each camber C1, C2 may be associated to a respective deviation angle a1, a2. Each deviation angle a1, a2 may be superior or equal to 15 or 25°.

The first camber C1 provided in outer face of the first clamping jaw 11 may be arranged between the first passage 31 and a first outer boss B of the clamping device. Such boss B is adjacent to the tongue 5. The second camber C2 provided in outer face of the second clamping jaw 12 may be located between the second passage 32 and the second outer boss B' provided in the second clamp end E2. With such cambers C1, C2, an expanding effect is obtained, allowing a greater sizing of the respective clamp ends E1, E2 while keeping the gap between these ends E1, E2 before actuating closure of the device 1, 1'.

Each of the clamps ends E1, E2 may optionally be provided with a loop profile, thus each having a hollow 17, 18, ZC. At least one hollow 17, ZC of the first clamp end E1 can open laterally outwards and the same can apply for a hollow 18 provided in the second clamp end E2. The second clamp end E2 can be more compact than the first clamp end E1. When hollows 17, 18 ZC are provided, the hollow 18 of the second clamp end E2 may be of lower circumference/perimeter than for hollow 17, ZC of the first clamp end E1.

**Embodiments with two-piece construction**

Referring to Figs 4-5 and 7, a clamping device 1' is illustrated with a construction using two complementary pieces. The two pieces are generally sized to each have:

- a half portion of the first clamp end E1 and a half portion of the second clamp end E2;
- a given fraction of the total width of the whole clamping device 1', at least in regions to form the clamping parts 8a, 8b.

Typically, the two pieces may each have same shape/profile (as viewed in a longitudinal plane), which is same general profile as a device 1 such as precedingly described.

Referring to Fig. 7a, one of the two pieces is illustrated, which is a piece 101 having male interlocking members protruding toward a same side, here right side when considering the bendable connection is at a rear side and the boss B with the pressing surface PS (in the first clamp end E1) is at a top of the device 1'. More generally, the piece 101 is adapted to be interlocked with a complementary piece 102, so that each of the two passages 31, 32 can be split, allowing insertion the hose 2 between the two pieces 101, 102 before an assembling of the clamping device 1'. The assembled configuration of these pieces 101, 102 may be locked in suitable manner, for instance using at least two interlocking members, forming protrusions 41, 42, 43, that are engaging complementary cavities/female parts (at least two of them), for instance using a clip connection in two or three distinct regions, possibly including a fastening inside the first clamp end E1 and inside the second clamp end E2.

As shown in Fig. 5, a longitudinal plane P, which is possibly a median plane or symmetry plane sharing the clamping device 1' in two halves having same width, may define a junction plane for the fastening between the two pieces 101, 102, here pieces that each form a loop (same general profile as in the first embodiment with a single piece construction). The features provided in the device 1 as above discussed may be identically present, possibly with the same structure, except that a junction is present for interconnecting the pieces 101, 102. Such pieces 101, 102 may be made separately. Possibly, a hinge connection may also be provided, for instance with the hinge arranged on a lower part of the device 1', for instance along a straight part of the second clamping jaw 12.

In another variant, a fastening of two parts may similarly obtained, but with a construction using a single piece, for instance using a living hinge, similar articulated connection, or using at least one flexible link.

Now referring to Fig. 6, it is shown a junction J' that may be offset from a symmetry plane, such junction being provided along a longitudinal plane intersecting the bendable connection 4 and the two clamp ends E1, E2. It thus understood that delimitations of the two passages 31, 32 are distributed in the two pieces 101, 102. As in the first depicted embodiment, the lips 9 are can be seen in a top view, with the second lip forming a boss B' that has its maxim thickness preferably provided at a top of the second clamp end E2, thus forming a large upper face F2 that may extend longitudinally (along a length direction of the device 1, 1') with an extension d2 that is more than 10 or 12% of the total length of the device 1, 1'. Such disposition may apply for any kind of production method used to make the clamping device, independently from the number of pieces involved. Extension d2 typically represents a maximum of thickness of the second clamp end E2 (possibly without taking the ribs 7 into account).

When having two halves or parts to be assembled, each boss B, B' may be an assembly of two complementary boss parts HB and HB' that have same circumference/profile and are joined along the junction J, J'. Each of the clamp ends E1, E2 may be entirely divided in two complementary parts, possibly forms in the two plastic pieces 101, 102. In some options, one of the clamping parts 8a or 8b can be formed selectively in one of the two pieces, while each of the two lips 9a, 9b is made of two separable lip parts distributed in the two pieces 101, 102. In preferred embodiments, the junction J, J' is typically a planar junction except at location of the interlocking members that form protrusions 41, 42, 43 (with a local protruding effect beyond the plane of the junction J, J').

In some options, at least one of the protrusions 41, 42, 43 are provided with radial tab, relief or annular bead (that protrudes outwardly), forming clips. Use of slanted reliefs maybe used to facilitate insertion of the protrusions, with a guiding effect. More generally, any assembling operation of two pieces can be performed to obtain the clamping device 1', so that the two pieces 101, 102 can be fastened quickly, using the interlocking elements (41, 42, 43, O1, O2, O3), knowing the flexible hose 2 is already extending between these two pieces 101, 102 before the fastening.

**Non-limiting exemplary use**

Fig. 3 depicts a fluid circuit 10, for instance a blood collection set of known type that is used in the collection of blood from a donor. A fluid circuit with clamping devices 1 could be also provided for circulation of another suitable biopharmaceutical product/process. Clamping devices 1 may be included in this circuit 10, for cooperation with one or more hoses 2a, 2b. Tubing set of the circuit 10 may include a venipuncture needle 14 and a length of tubing 200. The tubing 200 here branches at Y-connector 16 into a hose 2a and a hose 2b. The hose 2a provides a flow path to a collection container 100 and the hose 2b provides a flow path to a sampling pouch 20. The sampling pouch 20 may also include a holder 21 for receiving a blood sampling vial or tube (Also shown is a needle protector 15 for storing the needle after use). More generally, the clamping device 1, 1' may have a closed loop profile, while allowing a hose segment 2 to extend across an alignment direction of the device, such direction typically being an alignment direction of the two passages 31, 32.

In the blood tubing sets of the type shown in Fig. 3 or similar biopharmaceutical fluid circuits, segments 2a and 2b are passed through the two respective flow control clamping devices 1. The anchoring is performed in a region offset relative to the hose passageway, with a closed position obtained in response to a pressing portion directed toward the hose (radial pressing action). The securing or anchoring surface formed by the toothed section TS can typically extend radially, along a direction D1 transverse to the hose passageway as illustrated in particular in Figs 2 and 3. Passages 31, 32 thus may be considered as axial opposite openings in the clamping device 1 or 1'.

In some options, the clamping device 1, 1' may be configured so that, once closed, it remains irreversibly closed. An "irreversibly" closed or closable flow control clamp can only be released from the closed and locked position by extraordinary and unintended manipulation of the clamp, including breakage of the clamp.

For ease at exerting the pressing and displacing the tongue 5, the first clamp end E1 may include material that is robust, for instance a rigid or semi-rigid plastic. In some embodiments, at least one of the bosses B, B' is formed as a layer added on a plastic piece or support (that includes the tongue and the securing section and which possibly delimits an inner circumference of the clamping device in closed position), with the boss B and/or B' being welded, mechanically fastened or overmolded.

A hollow 17, 18 may optionally be formed between the plastic piece or support and the boss B, B' (optionally arranged as an added layer), allowing a good trade-off between obtention of a significant bulging effect at one or two of the lips 9a, 9b and optimization of plastic material included in the clamping jaws 11, 12.

In addition or in a variant, at least one boss B, B' is obtained as an assembly of two clamp end halves or two fastening parts, for instance with a fastening performed in a core part of the corresponding clamp end E1, E2. Preferably when two pieces are used, each piece 101, 102 may contain a single plastic material. Such use of a same plastic material can also apply when having a single-piece construction.

Each piece 101, 102 may include a camber adjacent to the half clamp end. For instance, Fig. 7a shows a camber HC1 in the piece/body half 101, between a connector section that is provided with one amongst the side protrusions and the rear passage section (for forming passage 31). Similarly, the other piece/half body 102 as illustrated in Fig. 7b may have a camber HC2 extending between the pressing surface portion of the other connector section (with knowledge that the two connector sections, once assembled, are configured as a connector including the first clamp end E1) and the corresponding rear passage section of this piece 102.

When having two clamp bodies/pieces 101, 102, the one or more protrusion(s) 41 arranged in the connector section of the piece 101 may form an insert adapted to be hidden inside the other piece 102, here to extend inside the receiving cavity or passage O1. With such insert engaged in such transverse passage O1, the protrusion (s) 41 extends inside the boss B, typically inside the portion of this boss which is complementary from the boss part HB in the connector of the first clamping jaw 11.

The clamping device 1, 1' may be operated without significant difference as compared with clamps providing a toothed section for engagement with a tongue at an opposite clamp end, while offering an increased level of safety, making it user-friendly.

Referring to Fig. 6, it can be seen the tongue 5 may have an elongated base b5 joining two opposite sides of the first clamp end E1. The tongue 5 can also be slanted and provided with a rectilinear edge that extends between two rounded side ends of the tongue 5. As a result, the rectilinear edge may be shorter than the elongated base b5 and risk of degradation or injury due to such edge at a side thereof is prevented. Such disposition can apply for any of the embodiments or options previously exposed about the clamping device 1 or 1'.

While embodiments are shown with each passage 31, 32 delimited by a circumference, without any deformation of such circumference, a clamping device 1, 1' may also be provided with one or two arched blades or similar flexible tabs, able to hold a hose of smaller size that an inner diameter or maximal size defined at one of the passages 31, 32. Possibly, the passage 31 may be provided with two flexible C-shaped branches that protrude from a same side of the passage circumference, with a divergent bending/ability to be bent outwardly: such pair of branches may prevent deviation or radial motion of a tube/hose of size smaller than the passage characteristic diameter.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

**Claims**

1. A clamping device (1; 1') for regulating a flow of liquid passing through a flexible hose (2), comprising a first clamping jaw (11) and a second clamping jaw (12) that are arranged for gripping the flexible hose (2) therebetween, using two passages (31, 32) that are distributed at rear and front ends of the clamping device and are forming a hose passageway for receiving the flexible hose,

        wherein the clamping device (1; 1') comprises:

            - two clamping parts (8a, 8b), between which a hose segment of the flexible hose passing through the two passages (31, 32) can be nipped in closed state of the clamping device (1), the two clamping parts (8a, 8b) being distributed in the first clamping jaw (11) and in the second clamping jaw (12);
- offset the hose passageway, two clamp ends (E1, E2) distributed in the two clamping jaws (11, 12) and provided with interacting securing elements that anchor the clamping jaws (11, 12) in a closing position,

wherein one of the two clamp ends (E2) includes a securing surface with at least one retaining rib or tooth (7) facing inwardly, while the other clamp end (E1) is actuatable in response to a pushing action and provided with a tongue (5) to engage with said securing surface, two outer lips (9) being provided at the two respective clamp ends (E1, E2),
and wherein in an expanded state of the clamping device (1; 1'), the tongue (5) is;

- transverse to the securing surface;
- protruding outwardly, while being housed entirely inside a mouth region delimited between the two outer lips (9).

2. The clamping device according to claim 1, forming a closed loop in the closed position, and an open loop in the expanded position,

wherein the two clamp ends comprise a first clamp end (E1) and a second clamp end (E2) that are adapted to constitute the two ends of said open loop,
and wherein the first clamping jaw (11) includes a first bulged end part provided with a first lip (9a) of the two outer lips that defines an outer free end of the first clamp end (E1), while the second clamping jaw (12) includes a second bulged end part provided with a second lip (9b) of the two outer lips that defines an outer free end of the second clamp end, the first lip (9a) preferably having a convex surface with a convex or rounded profile extending between the tongue (5) and a pressing surface (PS) of the first bulged end part.

3. The clamping device according to claim 1 or 2, wherein the securing elements consist of the tongue (5) and a toothed section (TS) that includes the securing surface, the securing elements allowing maintaining a closed state of the clamping device (1) when mutually engaged, and being adapted to anchor the clamping jaws (11, 12) in at least two closing positions,

and wherein the clamp end (E1) provided with the tongue (5) has a longitudinal axis (X1), a by-default interspace (e8) between the clamping parts (8a, 8b), obtained in the expanded state, being greater than any interspace delimited in any one of the closing positions, between the clamping parts, the tongue (5) being configured, in the expanded state, to protrude along the longitudinal axis (X1) that is transverse to the toothed section (TS).

4. The clamping device according to claim 3, wherein the two clamp ends comprise a first clamp end (E1), which is provided with the tongue (5), and a second clamp end (E2),

wherein the toothed section (TS) is facing inwardly,
and wherein the tongue (5) protrudes, in the expanded state, substantially perpendicular to a pressing direction allowing the first clamp end (E1) to be displaced and engaged onto said toothed section (TS) formed on an inner face of the second clamp end (E2).

5. The clamping device according to any one of the preceding claims, wherein in the expanded state of the clamping device (1), the tongue (5) extends at a rear of a tangential virtual plane (PT) tangent to the two outer lips (9) and protrudes along a general direction (D5) that virtually intersects the tangential virtual plane (PT).

6. The clamping device according to claim 5, wherein one of the two outer lips formed in the first clamping jaw (11) defines an uppermost portion of the clamping device (1; 1'), while the other one of two outer lips defines a front free end of the clamping device (1; 1') that protrudes forward, the virtual plane (PT) being a plane tangent to said uppermost portion and said front free end in the expanded state.

7. The clamping device according to any one of the preceding claims, wherein the first clamping jaw (11), provided above the second clamping jaw (12), extends from as a first passage (31) of the two passages,

wherein the second clamping jaw (12) also extends from the first passage (31) and has a second passage (32) of the two passages provided below the second clamp end (E2), with a direction of the two passages (31, 32) allowing the flexible hose (2) to extend on a same lower side relative to the two clamp ends (E1, E2),
and wherein the clamp end (E2) provided with the securing surface has a thickness which progressively increases with increased spacing from a transition portion of the second clamping jaw adjacent to the second passage (32), whereby a boss (B') is formed adjacent to the securing surface.

8. The clamping device according to any one of the preceding claims when depending on claim 2, wherein the two outer lips (9) have each a thickness superior to 7 mm, preferably 9 mm, as measured:

   - perpendicular to protruding general direction (D5) of the tongue (5), for thickness of the outer lip (9a) provided in the first clamp end (E1);
   - perpendicular to direction of extension (D1) of the securing surface, for thickness of the outer lip (9b) provided in the second clamp end (E2).

9. The clamping device according to any one of the preceding claims when dependent on claim 2, wherein the first clamping jaw (11) has an outer face provided with a first camber (C1) arranged between a first passage (31) amongst the two passages and a fist outer boss (B) of the clamping device that is adjacent to the tongue (5), and wherein the second clamping jaw (12) provides a second camber (C2) in an outer face of the second clamping jaw (12), the second camber (C2) being arranged between a second passage (32) amongst the two passages and a second outer boss (B') of the clamping device that is provided in the second clamp end (E2), facing in a direction opposite to the securing surface.

10. The clamping device according to any one of the preceding claims when dependent on claim 2, wherein the toothed section (TS) is provided in the second clamping jaw (12) and comprises teeth (7) distributed and mutually spaced along a given alignment direction (D1),

   and wherein the following relation is satisfied:

   $$0.3 \leq L1/L2 \leq 0;6$$

   where L1 is the spacing distance, measured along the alignment direction, between the toothed section (TS) and the tongue (5) in said expanded state, while L2 is the spacing distance, measured along the alignment direction (D1), between the toothed section (TS) and an outer surface portion formed in a boss of the first clamping jaw (11).

11. The clamping device according to any one of the preceding claims, comprising a bendable connection (4) that forms a common end for the first clamping jaw (11) and the second clamping jaw (12), which are thus bendably connected with each other at this common end, wherein the securing elements are provided in the two clamp ends (E1, E2), respectively at the opposite of the common end.

12. The clamping device according to claim 11, made of a single piece with the bendable connection (4) formed in a C-shaped portion that extends between the clamping parts (8a, 8b).

13. The clamping device according to claim 11, made of two pieces provided with interlocking elements, the bendable connection (4) and delimitations of the two passages (31, 32) being distributed in the two pieces, so that the two pieces can be fastened using the interlocking elements (41, 42, 43, O1, O2, O3) to obtain the clamping device (1') once the flexible hose (2) is already extending between the two pieces of the clamping device (1').

14. The clamping device according to claim 13, wherein at least one of the two lips (9), preferably each of the two lips, is made of two separable lip parts distributed in the two pieces.

15. The clamping device according to any one of the preceding claims, wherein the tongue (5) is:

   - provided with an elongated base joining two opposite sides of the clamp end (E1) included in the first clamping jaw (11);
   - slanted; and
   - provided with a rectilinear edge that extends between two rounded side ends of the tongue (5), so that the rectilinear edge is shorter than the elongated base of the tongue (5).

16. The clamping device according to any one of the preceding claims, wherein at least one amongst the two clamp ends (E1, E2) is provided with a hollow (ZC) that opens laterally outwards.

FIG. 1

FIG. 2

**FIG. 3**

**FIG. 4**

P
1'
41
101
102
D5
d2
8a
O1
O2
42
e8
a2
8b
31
43
O3
32

**FIG. 5**

B'
9
5
9
B
J'
4
102
31
101
1'
F2
e5
F1
11

**FIG. 6**

FIG. 7a

FIG. 7b

Europäisches
Patentamt

European
Patent Office

Office européen
des brevets

# EUROPEAN SEARCH REPORT

**Application Number**

EP 23 21 0170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/233832 A1 (TAPPEHORN LUDGER [DE] ET AL) 27 July 2023 (2023-07-27) | 1-12,15 | INV.<br>A61M39/28<br>F16K7/06 |
| Y | * figures 2-4 *<br>* paragraphs [0092] – [0109] *<br>----- | 13,14 | |
| X | US 2009/306619 A1 (MATHIAS JEAN-MARIE [BE] ET AL) 10 December 2009 (2009-12-10) | 1,2, 5-12,15 | |
| Y | * figures 1-11 *<br>* paragraphs [0028] – [0040] *<br>----- | 13,14 | |
| X | US 8 474 784 B2 (KASHMIRIAN AVTAR SINGH [AU]; DALLAPIAZZA JOSEPH J [US] ET AL.) 2 July 2013 (2013-07-02) | 1,2, 5-12,15, 16 | |
| Y | * figures 11, 12 *<br>* column 6, lines 18-56 *<br>----- | 13,14 | |
| X | US 6 644 618 B1 (BALBO ENRICO [IT]) 11 November 2003 (2003-11-11) | 1-12 | |
| Y | * figures 1-3 *<br>* column 2, line 11 – column 3, line 12 *<br>----- | 13,14 | **TECHNICAL FIELDS SEARCHED (IPC)** |
| Y | US 2008/082079 A1 (BRAGA RICHARD [US] ET AL) 3 April 2008 (2008-04-03)<br>* figures 2, 9, 9A, 9B, 10A *<br>----- | 13,14 | F16K<br>A61M |

The present search report has been drawn up for all claims

| Place of search | Date of completion of the search | Examiner |
|---|---|---|
| Munich | 4 April 2024 | López García, Mónica |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................

& : member of the same patent family, corresponding document

EPO FORM 1503 03.82 (P04C01)

EP 4 556 051 A1

## ANNEX TO THE EUROPEAN SEARCH REPORT
## ON EUROPEAN PATENT APPLICATION NO.

EP 23 21 0170

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2024

| Patent document cited in search report | | Publication date | Patent family member(s) | | Publication date |
|---|---|---|---|---|---|
| US 2023233832 | A1 | 27-07-2023 | DE 102023100948 | A1 | 27-07-2023 |
| | | | US 2023233832 | A1 | 27-07-2023 |
| US 2009306619 | A1 | 10-12-2009 | AT E480293 | T1 | 15-09-2010 |
| | | | EP 1469904 | A1 | 27-10-2004 |
| | | | EP 2357016 | A1 | 17-08-2011 |
| | | | US 2005215975 | A1 | 29-09-2005 |
| | | | US 2009306619 | A1 | 10-12-2009 |
| | | | US 2012172755 | A1 | 05-07-2012 |
| | | | US 2015133876 | A1 | 14-05-2015 |
| | | | US 2017100578 | A1 | 13-04-2017 |
| | | | US 2017361086 | A1 | 21-12-2017 |
| | | | WO 03063945 | A1 | 07-08-2003 |
| US 8474784 | B2 | 02-07-2013 | AU 2007233576 | A1 | 11-10-2007 |
| | | | CA 2643595 | A1 | 11-10-2007 |
| | | | EP 2007474 | A1 | 31-12-2008 |
| | | | JP 5255560 | B2 | 07-08-2013 |
| | | | JP 2009532137 | A | 10-09-2009 |
| | | | US 2010096570 | A1 | 22-04-2010 |
| | | | WO 2007112500 | A1 | 11-10-2007 |
| US 6644618 | B1 | 11-11-2003 | AU 5564100 | A | 02-01-2001 |
| | | | IT 1310919 | B1 | 27-02-2002 |
| | | | US 6644618 | B1 | 11-11-2003 |
| | | | WO 0077428 | A2 | 21-12-2000 |
| US 2008082079 | A1 | 03-04-2008 | CA 2602300 | A1 | 28-03-2008 |
| | | | EP 1905478 | A2 | 02-04-2008 |
| | | | US 2008082079 | A1 | 03-04-2008 |
| | | | US 2012203187 | A1 | 09-08-2012 |
| | | | US 2014128820 | A1 | 08-05-2014 |

EPO FORM P0459

For more details about this annex : see Official Journal of the European Patent Office, No. 12/82

**REFERENCES CITED IN THE DESCRIPTION**

**Patent documents cited in the description**

- US 6196519 B
- US 6644618 B
- US 8801677 B